# FASCICULE DE BREVET EUROPEEN

(11) **EP 0 451 299 B1**
(45) Date de publication et mention de la délivrance du brevet: **19.04.1995**
(21) Numéro de dépôt: 90917517.6
(22) Date de dépôt: 16.10.1990
(51) Int. Cl.: A61K 47/02, A61K 31/71, A61K 38/43, A61K 31/135, A61K 33/26, A61K 33/44

(54) **SUPPORT A COMMANDE MAGNETIQUE A SUBSTANCE PHYSIOLOGIQUEMENT ACTIVE ET METHODE DE REALISATION DUDIT SUPPORT**
MAGNETOKONTROLLIERBARER TRÄGER MIT PHYSIOLOGISCH AKTIVEN MITTELN UND VERFAHREN ZU SEINER HERSTELLUNG
MAGNETOCONTROLLABLE CARRIER WITH PHYSIOLOGICALLY ACTIVE SUBSTANCE AND METHOD FOR MAKING SAID CARRIER

(30) Priorité: 02.11.1989 SU 4767768
(43) Date de publication de la demande: 16.10.1991
(73) Titulaire: KHOLODOV, Leonid Efimovich, Moscow, 117630 (SU); VOLKONSKY, Viktor Alexeevich, Moskovskaya obl. pos. Aprelevka, 143360 (SU); KOLESNIK, Nikolai Fedorovich, Zaporozhie, 330096 (SU); PRILUTSKY, Oleg Volfovich, Zaporozhie, 330006 (SU); SEDOV, Alexandr Nikolaevich, Zaporozhie, 330006 (SU); KUZNETSOV, Anatoly Alexandrovich, Moscow, 117333 (SU); FILIPPOV, Viktor Ivanovich, Moscow, 117511 (SU); LEPAKHIN, Vladimir Konstantinovich, Moscow, 123007 (SU); BELOUSOV, Jury Borisovich, Moscow, 117321 (SU)
(72) Inventeur: KHOLODOV, Leonid Efimovich, Moscow, 117630 (SU); VOLKONSKY, Viktor Alexeevich, Moskovskaya obl. pos. Aprelevka, 143360 (SU); KOLESNIK, Nikolai Fedorovich, Zaporozhie, 330096 (SU); PRILUTSKY, Oleg Volfovich, Zaporozhie, 330006 (SU); SEDOV, Alexandr Nikolaevich, Zaporozhie, 330006 (SU); KUZNETSOV, Anatoly Alexandrovich, Moscow, 117333 (SU); FILIPPOV, Viktor Ivanovich, Moscow, 117511 (SU); LEPAKHIN, Vladimir Konstantinovich, Moscow, 123007 (SU); BELOUSOV, Jury Borisovich, Moscow, 117321 (SU)
(74) Mandataire: Hirsch, Marc-Roger
(86) Numéro de dépôt international: SU9000237
(87) Numéro de publication internationale: WO9106322

(56) Documents cités:
- AU-B-37 610 /78
- DE-A- 3 007 878
- GB-A- 2 035 798
- US-A- 4 183 918
- US-A- 4 269 826

## Description

La présente invention qui concerne le domaine de la médecine a notamment pour objet un support à commande magnétique porteur d'une matière physiologiquement active et un procédé d'obtention de ce support.

On connaît actuellement des supports à commande magnétiques variés, utilisés pour le transport ciblé des matières physiologiquement actives sous l'effet d'un champ magnétique et les procédés d'obtention de ces supports. On connaît, par exemple, des microcapsules (microemboles) magnétiques sous enveloppe de polymère et leur procédé d'obtention (Sako M, Hirota S. -Can To Kagaku Rycho, 1986, 13, N° 4, Pt 2, p. 1618-1624). Ces microcapsules magnétiques (d'une taille de 30 à 50 »m) sont utilisées dans le cas d'embolie de vaisseaux sanguins irrigant une tumeur. Elles sont introduites sous forme d'une suspension visqueuse en solution aqueuse de polysaccharides (dextrane à 12-40%) de sel sodique à 2 % de carboxycellulose et de préparations anticancéreuses.

On connaît également des microcapsules à commande magnétique porteuses de matières physiologiquement actives, contenant de la ferrite de zinc en tant que substance ferromagnétique (Kato H. et al., Appl. Biochem. Biotechnol., 1984, 10, p. 199-221) ou des microcapsules se composant d'un noyau ferromagnétique et d'une préparation anticancéreuse (mitomycine C); on utilise alors l'éhtylcellulose comme matériau formant enveloppe (Sugibarbik et al., J. Biomaterials, 1982, v. 3, p. 181-186).

On connaît un procédé d'obtention de liposomes (Farmakologia i toksikologia, Moscou, 1985, v. 48, N° 5, p. 32-35) contenant des particules ferromagnétiques d'une taille d'environ 200 mm. Comme matières physiologiquement actives ces particules contiennent des médicaments curariformes (pyrocurine, diadonium) et leur enveloppe est formée de polysaccharides.

Tous les supports à commande magnétique précédemment cités se caractérisent par une faible activité biologique du fait de leur mauvaise tenue dans un champ magnétique permanent créé au niveau d'un foyer pathologique, de sorte que ces supports ainsi que les matières physiologiquement actives qu'ils portent sont entraînés en grande partie par la circulation sanguine, du fait de la faible sensibilité magnétique du matériau ferromagnétique utilisé.

Les supports connus se caractérisent par une activité biologique limitée des matières physiologiquement actives du fait d'une perméabilité de l'enveloppe difficile à régler, de son épaisseur variable et de sa mauvaise stabilité au stockage. Ces supports sont difficiles à fabriquer et à stériliser sans altération de leurs propriétés.

On connaît également des microsphères contenant un matériau ferromagnétique et une matière physiologiquement active sous enveloppe albumineuse, et leur procédé d'obtention (Widder K.J., et al., J. Pharm. Sci., 1979, 68, p. 79-82). On utilise de la magnétite (Fe₃O₄) comme matériau ferromagnétique, une préparation anticancéreuse (doxorubicyne) comme matière physiologiquement active et de l'albumine comme matériau formant enveloppe. La taille des microsphères est voisine de 1 »m. Le procédé d'obtention des microsphères consiste à dénaturer l'albumine à haute température, ou chimiquement, en émulsion (eau dans l'huile de coton). La phase précipitée aqueuse se présente alors sous forme de gouttelettes d'eau d'une taille d'environ 1 »m auxquelles il faut incorporer des microparticules de magnétite, et qui doivent contenir en solution l'albumine et le médicament. L'émulsion est obtenue par un traitement aux ultra-sons avant et pendant la dénaturation, puis on sépare les microsphères par centrifugation, on les lave soigneusement à plusieurs reprises pour les débarrasser de l'huile, et on les fait sécher. Ces microsphères se caractérisent par une toxicité élevée du fait qu'elles contiennent de l'albumine dénaturée qui représente un corps étranger vis-à-vis de l'organisme, par une activité biologique limitée des matières physiologiquement actives du fait des variations de l'épaisseur de l'enveloppe, et par une altération de la perméabilité de l'enveloppe pendant le stockage. En outre, l'activité des matières physiologiquement actives contenues dans les microsphèrs vis-à-vis des foyers pathologiques, et donc l'efficacité du traitement, est limitée par la faible sensibilité magnétique des particules dispersées du fait de la teneur relativement faible des microsphères en matériau ferromagnétique (en magnétite), de sorte que les particules ferromagnétiques porteuses de la matière physiologiquement active ne sont pas stables dans le champ magnétique permanent créé au niveau d'un foyer pathologique et, qu'une grande partie des microsphères portant la matière physiologiquement active est emportée par la circulation sanguine. Le procédé d'obtention de ces microsphères est difficile à réaliser. Les microsphères sont instables pendant le stockage ("vieillissement" de l'enveloppe, agglomération).

Dans le cadre de l'invention on s'est proposé de créer un support à commande magnétique porteur d'une matière physiologiquement active, caractérisé par une grande activité biologique et une faible toxicité, et de simplifier le procédé de son obtention.

Ce problème est résolu grâce à un support à commande magnétique porteur d'une matière physiologiquement active constitué, selon l'inventioin, par des particules de fer-carbone composées de 10 à 90 % en masse de fer et de carbone pour le reste, ayant une capacité d'adsorption d'au moins 6 mg/g pour le bleu de méthylène et portant par adsorption la matière physiologiquement active.

Selon l'invention, les variétés les plus souhaitables de support à commande magnétique porteur d'une matière physiologiquement active sont les suivantes :
- support à commande magnétique composé de 50 à 70 % en masse de fer, et de carbone pour le reste, et portant de 2 à 2,5 % en masse d'antibiotique anticancéreux anthracyclinique (daunorubicine, doxorubicine ou carminomycine) ;
- support à commande magnétique composé de 50 à 70 % en masse de fer, et de carbone pour le reste, et portant une enzyme (célyase) dans une proportion de 500 UI/mg de particules de fer-carbone ;
- support à commande magnétique composé de fer à 50-70% en masse, et de carbone pour le reste, et portant une préparation antihistaminique (diphénhyldramine) dans une proportion allant de 2 à 3 % en masse.

Le support à commande magnétique porteur d'une matière physiologiquement active selon l'invention permet de multiplier l'activité biologique par 2 par rapport aux microsphères connues pour les mêmes doses des matières physiologiquement actives, et de multiplier par 2 jusqu'à 6 l'activité biologique par rapport aux formes médicamenteuses usuelles des matières physiologiquement actives administrées dans les doses maximales possibles. Le support à commande magnétique, porteur d'une matière physiologiquement active, selon l'invention, est peu toxique et ne comporte pas (contrairement aux microsphères connues) d'albumine dénaturée étrangères vis-à-vis de l'organisme.

Grâce à la forte liaison entre le carbone le métal, les particules fer-carbone du support selon l'invention permettent de maintenir de manière sûre les matières physiologiquement actives au niveau d'un foyer pathologique sous l'action d'un champ magnétique et d'éviter ainsi leurs pertes dans l'organisme. La désorption des matières physiologiquement actives depuis la surface des particules de fer-carbone (contraîrement à la diffusion des matières physiologiquement actives à travers une enveloppe) permet aux matières physiologiquement actives d'accéder à un foyer pathologique à une vitesse pratiquement constante, alors que la vitesse de diffusion à travers une enveloppe varie du fait des caractéristiques variables de l'enveloppe elle-même.

Le procédé d'obtention du support à commande magnétique porteur d'une matière physiologiquement active selon l'invention, consiste à chauffer le fer en poudre dans un milieu oxydant à une température de 800 à 1200°C, puis à le traiter avec du monoxyde de carbone à une température de 400 à 700°C pour obtenir les particules de fer-carbone, et à les introduire dans une solution de matières physiologiquement actives, où ces matières sont adsorbées par la surface desdites particules. La taille des particules de fer en poudre est de préférence comprise entre 100 et 500 »m. Il est également préférable d'utiliser l'air ou la vapeur d'eau comme milieu oxydant.

Le procédé selon l'invention permet d'obtenir de façon peu complexe un support à commande magnétique porteur d'une matière physiologiquement active caractérisé par une grande activité biologique. Grâce au traitement à haute température, les particules de fer-carbone sont stériles et peuvent être soumises, si nécessaire, à une stérilisation supplémentaire, sans que cela nuise à leurs caractéristiques sur le plan de la qualité.
La meilleure variante de réalisation.

Le support à commande magnétique porteur d'une matière physiologiquement active selon l'invention se présente sous forme d'une poudre finement dispersée de couleur noire attirable par un aimant, et se compose de particules de fer-carbone (d'une taille allant de 0,05 à 0,5 »m pour la plupart) dont le noyau en fer réduit est associé de manière solide aux particules allongées de carbone active (ayant l'aspect de cristaux filiformes) caractérisées par une grande résistance mécanique spécifique et une haute capacité d'adsorption. C'est la surface de ces particules qui adsorbe la matière physiologiquement active. La teneur en fer des particules de fer-carbone est de 10 à 90 % en masse, le reste étant du carbone.

Une teneur en fer plus faible (inférieure à 10 % en masse) et, donc une teneur en carbone plus grande, ne permet pas d'obtenir l'activité biologique voulue aux matières physiologiquement actives, car la sensibilité magnétique diminue du fait de la diminution de la proportion du constituant métallique du support. Une teneur en fer plus forte (plus de 90 % en masse) et une teneur en carbone plus faible provoquent une réduction de la capacité d'adsorption, et donc une diminution de la quantité de matière physiologiquement active adsorbée.

Selon l'usage auquel elles sont destinées, les matières physiologiquement actives adsorbées peuvent être variées : anticancéreuses et antibiotiques, moyen pharmacologiques, par exemple, préparations antihistaminiques, substances albumineuses, par exemple, les enzymes.

Le support à matière physiologiquement active selon l'invention a fait l'objet d'études expérimentales portant sur les animaux ainsi que des essais cliniques chez les hommes.

Dans les essais effectués, le support selon l'invention était porteur d'une préparation anticancéreuse : daunorubicyne, doxorubicyne ou carminomicyne ; teneur en fer : 56 à 64 % en masse, le reste étant du carbone ; teneur en antibiotique daunorubicyne, doxorubicyne ou carminomycine adsorbé : 2 à 2,5 % en masse.

L'action anticancéreuse a été établie chez des rats et comparée à celle des microsphères connues portant lesdits antibiotiques (taille moyenne des microsphères: 0,2 à 0,5 »m ; enveloppe en albumine dénaturée), ainsi qu'à celle de ces médicaments anticancéreux sans support à commande magnétique. Les sujets expérimentaux étaient des rats d'une masse allant de 180 à 220 g. On a réinoculé aux rats (au milieu de la queue) une des deux couches des tumeurs réinoculables des rats ; carcinosarcome de Walker, sarcome S-45 ou sarcome M-1 (40 rats pour chaque souche). Le traitement des animaux a commencé lorsque le volume de la teneur avait 1000 mm³.

Le support selon l'invention contenant de la daunorubicyne ou de la carminomicyne a été injecté en une seule fois lentement dans la veine caudale des rats, dose de 40 mg (0,8 mg en calculant en antibiotique, soit 4 mg/kg), sous forme d'une suspension en 1 ml de solution de gélatinol à 8 % pour injections. Dans le cas de la doxorubicyne la dose a été de 12 mg/kg (dose de support : 96 mg en calculant en antibiotique. Avant l'injection, un aimant permanent de samarium-cobalt (6000 Oe) a été fixé sur la queue au niveau de la tumeur. La localisation du support à antibiotique selon l'invention dans le vaisseau au niveau de la tumeur a été contrôlée à l'aide de radiogrammes.

Les microsphères albumineuses ont été injectées en une seule fois, comme décrit ci-dessus, dans une dose de 4 mg/kg (en calculant en daunorubicyne ou en carminomicyne). Les antibiotiques (sans support à commande magnétique) ont été injectés comme suit : la daunorubicyne a été injectée dans la veine caudale des rats avec une dose de 2 mg/kg/jour en solution de gélatinol à 8 % pour des injections pendant 5 jours (une injection par jour) ; carminomicyne : 0,4 mg/kg 3 fois avec des intervalles de 120 heures ; doxorubicyne : en une seule fois avec une dose de 12 mg/kg. Dans le groupe témoin des animaux atteints de tumeur on a injecté dans la veine, 1 ml de solution de gélatinol à 8 % pour injections.

A la suite d'un seul traitement avec le support selon l'invention contenant la daunorubicyne, la doxorubicyne ou la carminomicyne, après sa localisation à l'aide d'un champ magnétique permanent de 6000 Oe au niveau de la tumeur (carinosarcome de Walker, sarcome S-45 ou sarcome M-1), on a constaté la régression complète de la tumeur dans 60 % des cas. Pendant toute la période d'observation (100 jours) on n'a pas constaté de récidives de la maladie chez des rats après résorption des tumeurs. Chez le reste des animaux soumis au traitement avec le support à antibiotiques selon l'invention, on a constaté un ralentissement considérable de la croissance de la tumeur et des zones de sa destruction ; la durée de vie de ces animaux a augmenté de 1,5 à 2 fois par rapport à celle des animaux du groupe témoin (sans antibiotiques).

Les examens histologiques ont démontré l'absence totale de cellules tumorales dans les échantillons de tissu prélevés dans la zone d'emplacement de la tumeur après sa résorption complète sous l'action du support, selon l'invention, à la daunorubicyne, la doxorubicyne ou la carminomycyne. Par des examens histologiques des échantillons de tissu du foie et des autres organes vitaux, aucune altération toxique qui caractérise l'emploi de ces antibiotiques sous forme de solutions ordinaires n'a été constatée. Chez les animaux traités de ce groupe on n'a noté aucune oppression de l'hémopoïèse ; en particulier, il n'y avait ni leucopénie ni thrombocytopénie.

En cas de traitement avec des microsphères albumineuses contenant les antibiotiques (4 mg/kg de daunorubicyne ou de carminomicyne) avec le même mode de localisation sous l'effet d'un champ magnétique analogue, on a constaté la régression complète de la tumeur chez 30 % des animaux. Chez les autres 30 à 40 % des animaux, la croissance de la tumeur s'est ralentie et la durée de vie a été multipliée par 1,4 fois par rapport au groupe témoin (sans antibiotiques). Chez le reste des animaux, le traitement aux microsphères n'a eu aucun effet thérapeutique. Chez une partie des animaux, on a constaté de la leucopénie comme résultat de l'effet opprimant des antibiotiques sur l'hémopoïèse.

Le traitement pendant 5 jours par la daunorubicyne (2 mg/kg/jour) ou par la carminomicyne (3 injections dans la dose de 0,4 mg/kg avec des intervalles de 120 h) n'a donné en aucun cas la régression complète de la tumeur, la durée de vie des animaux a augmenté de 15-30% en moyenne par rapport au groupe témoin (sans antibiotiques). Les examens histologiques de tous les animaux ont démontré des altérations cytotoxiques graves de divers tissus, surtout du tissu hépatique. Dès le 2-ème ou 3-ème jour de traitement on a constaté chez les animaux une oppression de l'hémopoïèse (la leucopénie et, chez une partie des rats, la thrombocytopénie).

Tous les animaux du groupe témoin (sans antibiotiques) sont morts.

Ainsi, le traitement à l'aide du support selon l'invention contenant la daunorubicyne, la doxorubicyne ou la carminomicyne permet d'obtenir dans 60 % des cas la disparition totale de la tumeur par l'injection intraveineuse d'une dose unique de médicament, et sa localisation dans la zone tumorale à l'aide du champ magnétique. Ce support est 2 fois plus efficace que les microsphères albumineuses connues pour une même dose d'antibiotique (30 % des cas de guérison), et 4 à 6 fois plus efficace (en ce qui concerne la durée de vie) que l'injection intraveineuse des solutions ordinaires de ces antibiotiques avec la dose de traitement maximale. En outre, contrairement aux microsphères ou aux solutions d'antibiotiques, l'emploi du support selon l'invention ne conduit pas à une lésion toxique des tissus, y compris du tissu hépatique, et ne provoque aucune oppression de l'hémopoïèse.

On a effectué des essais de l'activité thrombolytique du support selon l'invention contenant l'enzyme (célyase) qui active le plasminogène (qui est une proenzyme fibrinolytique) contenu dans le sang : le plasminogène se transforme en plasmine, pénètre dans un thrombus et le dissout.

Le support selon l'invention est composé à 60 % de fer, et de carbone pour le reste ; la teneur en enzyme adsorbé (célyase) est de 50 UI par 1 mg de support.

Des essais comparatifs de l'activitgé thrombolytique ont été faits en la comparant à celle de la célyase injectée sous forme de solution ordinaire.

Les sujets des essais étaient des lapins mâles de l'espèce chinchilla d'un poids de 2,5 à 3 kg. Une section de 3 mm de diamètre de la paroi vasculaire de la veine marginale de l'oreille du lapin a été découpée et un thrombus s'est formé dans la zone d'altération du vaisseau qui restait partiellement perméable.

Le support selon l'invention contenant 500 UI de célyase par 1 mg a été injecté en une seule fois, lentement, avec une seringue dans la veine marginale de l'oreil du lapin (10 animaux) en amont du thrombus avec une dose de 40 mg (soit 20000 UI de célyase, soit 6,7·10³ à 8 000 MI/kg) sous forme de suspension dans 0,6 ml de solution de gélatinol à 9 % pour injections. Avant l'injection, un aimant permanent de samarium-cobalt (6000 Oe) a été fixé sur l'oreille dans la zone du thrombus. La localisation du support, selon l'invention, porteur de l'enzyme dans le canal vasculaire a été contrôlée par radiographie.

Dans le groupe d'animaux témoins (10 animaux), un thrombus a été obtenu dans la veine marginale de l'oreille, comme indiqué ci-dessus, et on a injecté dans cette veine en amont du thrombus une seule dose de célyase de 250000 UI dans 1 ml de solution de gélatinol à 8 % pour injections. L'effet thrombolytique a été évalué d'après l'hémorragie de la zone d'altération du vaisseau.

30 à 50 minutes après l'injection du support, selon l'invention, contenant la célyase (dose : 6,7 à 8 000 UI/kg) et sa localisation dans la zone du thrombus à l'aide de l'aimant permanent, on a constaté la lyse du thrombus qui obstruait la zone de lésion de la veine, ce qui a provoqué l'hémorragie de la zone d'altération du vaisseau. Le temps de coagulation d'un échantillon de sang prélevé sur la veine de l'autre oreille des lapins n'a pratiquement pas varié.

Dans une expérience témoin qui consistait à essayer de traiter un thrombus de la veine marginale de l'oreille des lapins par injections intraveineuse de la solution ordinaire de célyase avec une dose de 12,5 fois plus grande (250 000 UI/kg par lapin, soit 100 000 UI/kg), on a constaté seulement une dissolution partielle des parties périphériques du thrombus.

Ainsi, le traitement par le support, selon l'invention, contenant la célyase provoque la résorption (la lyse) du thrombus après une seule injection intraveineuse du support, selon l'invention, contenant la célyase (dose en calculant en enzyme près de 8000 UI/kg), et sa localisation dans la zone du thrombus par le champ magnétique. L'emploi d'une dose 12,5 fois plus grande (près de 100 000 UI/kg) de célyase sous forme de solution ordinaire ne permet d'obtenir qu'une dissolution peu significative des parties périphériques du thrombus. Ainsi, l'emploi du support, selon l'invention, contenant la célyase augmente de plusieurs fois l'activité thrombolytique locale en cas d'injection systématique de cette enzyme.

On a essayé l'activité anti-oedémique du support, selon l'invention, contenant une préparation antihistaminique (la diphénylhydramine). Le support selon l'invention se composait de 56 à 63 % en masse de fer et de carbone pour le reste ; la teneur en diphénylhydramine adsorbée était de 3 % en masse.

Des essais comparatifs de l'activitié anti-oedémique ont été effectués en la comparant à celle de la diphénylhydramine injectée sous forme de solution ordinaire.

Les sujets des essais étaient 30 rats mâles de la population Vistar d'un poids allant de 180 à 220 g. Pour obtenir un oedème expérimental, on a injecté par voie subplantaire au milieu de la queue de 10 rats, 0,1 ml de solution aqueuse de dextrane à 4 %. Un aimant permanent de samarium-cobalt (6000 Oe) a été fixé à cet endroit et on a injecté aussitôt lentement dans la veine caudale 20 mg de support, selon l'invention, contenant 0,6 mg de diphénylhydramine en 0,7 ml de solution de gélatinol à 8 % pour injections (dose moyenne en calculant en diphénylhydramine : 3 mg/kg). La localisation de la dispersion dans le canal vasculaire a été contrôlée par radiographie. Chez 10 rats on a injecté de la même façon la solution de dextrane et on a injecté aussitôt dans la veine caudale la solution de diphénylhydramine (dose : 20 mg/kg, soit en moyenne 5 mg par rat) en 0,7 ml de solution de gélatinol à 8 % pour injections. Chez 10 rats (groupe témoin) on a injecté de la même façon la solution de dextrane et on a injecté dans la veine 0,7 ml de solution de gélatinol à 8 % pour injections (sans médicament).

L'effet anti-oedémique a été évalué à l'aide d'un pléthysmomètre d'après la variation du volume de la queue par rapport aux animaux du groupe témoin.

Les résultats des essais ont démontré que dans le groupe témoin, 30 minutes après l'injection de dextrane, un oedème local de la queue s'est développé qui continuait à augmenter pendant toutes les 5 heures d'observation ; le volume de la queue 1 heure après l'injection de dextrane, a augmenté de 1535±52 mm ³. Dans le groupe traité par la solution ordinaire de diphénylhydramine introduite dans la veine avec une dose de 20 mg/kg l'oedème était légèrement moins prononcé : 1 heure après l'injection de la préparation (moment de l'effet anti-oedémique maximal) l'augmentation du volume de la queue était de 1025±41 mm³, soit de 33±2 % plus faible que dans le groupe témoin. Après le traitement par le support, selon l'invention, contenant la diphénylhydramine (20 mg, dose de diphénylhydramine : 0,6 mg, soit 3 mg/kg) injecté dans la veine caudale et localisé par le champ magnétique dans la zone d'injection de dextrane, l'oedème était à peine visible : le volume de la queue a augmenté de 355±31 mm³, soit une augmentation de 77±4 % plus faible que dans le groupe témoin.

Ainsi, l'emploi du support selon l'invention, contenant la diphénylhydramine et localisé par le champ magnétique dans le canal vasculaire dans la zone de développement d'un oedème provoqué par le dextrane exerce un effet-antioedémique de 2-2,5 fois plus fort que celui d'une dose nettement plus grande (de 7 fois) de diphénylhydramine injectée dans la veine sous forme de solution ordinaire.

On a testé la toxicité du support, selon l'invention, sur des rats et sur des souris dans le cas d'injection intraveineuse. Les résultats des essais ont démontré que DL₅₀ du support, selon l'invention, injecté par voie intraveineuse est de 3400 mg/kg de masse pour les rats, et de 3300 mg/kg de masse pour les souris.

Les essais ont démontré que le support selon l'invention (ne contenant pas de matières physiologiquement actives) exerce un effet anticancéreux considérable : une injection intravasculaire de ce support à des rats atteints du sarcome S-45 (160 mg/kg avec localisation magnétique dans la zone de la tumeur) augmente de façon certaine de 54 % en moyenne la durée de vie des animaux atteints de la tumeur. L'injection intravasculaire chez les rats du support, selon l'invention, contenant un médicament anticancéreux (la carminomicyne) diminue nettement la toxicité de la carminomicyne : la DL₅₀ est de plus de 25 mg/kg (en calculant en antibiotique) pour les rats, et de 18 mg/kg pour les souris, ainsi une toxicité est respectivement de 30 et de 10 fois plus faible que celle de la solution aqueuse de carminomicyne injectée par voie intraveineuse.

On arrive, en outre à diminuer de 1,5-2 fois la cardiotoxicité caractérisant l'antibiotique, et à affaiblir nettement l'effet opprimant de l'antibiotique sur l'hémopoïèse. Contraitement à la solution aqueuse de la carminomicyne, le support, selon l'invention, contenant la carminomicyne n'augmente par la coagulabilité du sang.

Le support selon l'invention porteur d'un antibiotique anticancéreux (carminomicyne) a été essayé en clinique. Le support selon l'invention se compose de 56-63% en masse de fer et de carbone pour le reste ; la teneur en carminomicyne adsorbée est de 2 à 2,5% en masse. Les essais cliniques consistaient à traiter un sarcome de la fesse au stade final (diamètre de la tumeur 12-15 cm). Une dose unique de 20 mg (en carminomicyne) du support, selon l'invention , contenant la carminomicyne a été injectée dans la veine assurant l'alimentation de la tumeur et, on a localisé le support au niveau de la tumeur à l'aide d'un champ magnétique. Au 3-ème jour la tumeur commençait à se résorber (selon les résultats de la radiographie). Au 8-ème jour on a effectué le drainage de la tumeur et on a aspiré les résidus de cellules de la zone de la nécrose. Ni une oppression de l'hémopoïèse ni des manisfestations de la cardiotoxicité n'ont été constatées. On a effectué l'hémosorption (deux fois). Le sujet se sentait bien. La concentration d'antibiotique dans le sang pendant 9 jours après l'injection uniques est stabilisée à un niveau de 3 à 6 ng/ml en moyenne. La concentration en médicament dans un échantillon de la tumeur prélevé par ponction au 8-ème jour a été 10 fois plus grande que dans le sang.

Le procédé d'obtention du support à commande magnétique porteur d'une matière physiologiquement active selon l'invention est le suivant.

Le fer en poudre est chauffé en atmosphère oxydante à une température de 800 à 1200°C. La granulométrie du fer en poudre est de préférence de 100 à 500 »m. Comme atmosphère oxydante on peut utiliser la vapeur d'eau, l'air ou un mélange gazeux oxygéné. On utilise de préférence l'air ou la vapeur d'eau. Le chauffage en atmosphère oxydante à cette température a pour effet qu'il se produit l'activation des particules ferromagnétiques et qu'il se forme à leur surface une pellicule d'oxydes de fer qui est d'une grande activité catalytique par rapport au monoxyde de carbone. Les particules ferromagnétiques activées sont ensuite traitées par le monoxyde de carbone à une température de 400 à 700°C, pour obtenir des particules de fer-carbone. Le carbone commence à se dégager à la surface catalytiquement active des particules ferromagnétiques au cours de leur traitement par le monoxyde de carbone.

Ce processus se caractérise en ce que les particules de carbone ainsi formées sont associées de façon solide au fer, et se présentent sous forme de cristaux filiformes. Les particules de fer-carbone ainsi obtenues sont d'une grande résistance mécanique spécifique et d'une grande capacité d'adsorption. Les particules de fer-carbone obtenues sont placées dans une solution de matières physiologiquement actives (solution dans l'eau ou dans un solvant organique) et dispersées par n'importe lequel des procédés connus (secouement, agitation ou traitement par ultra-sons d'une fréquence de 22 à 44 kHz pendans 5 à 30 minutes). Le constituant carbonique des particules de fer-carbone adsorbe alors de manière active les molécules des particules physiologiquement actives, alors que le constituant métallique permet d'obtenir un déplacement dirigé du médicament vers la zone d'action d'un champ magnétique permanent appliqué au foyer pathologique. La dispersion obtenue est séparée par centrifugation, par exemple, pour obtenir soit le produit visé, soit le support à commande magnétique portant adsorbée une matière physiologiquement active.

Le produit visé peut être obtenu sous forme de substance sèche ou resuspendu dans une solution visqueuse adaptée à l'emploi médical.

Le chauffage du fer en poudre en atmosphère oxydante est fait dans une gamme de températures de 800 à 1200°C.

Si la température de l'oxydation activante est inférieure à 800°C, il en résulte une diminution de l'activité catalytique de la poudre de fer et une réduction notable de la quantité du carbone qui s'y dégage, ce qui conduit à une diminution de la capacité d'adsorption et entraîne la diminution de l'activité des matières physiologiquement actives sur le foyer pathologique. Une température de traitement oxydant supérieure à 1200°C est également à déconseiller, car elle augmenterait les dépenses d'énergie et nécessiterait un équipement spécial.

Une diminution (ainsi qu'une augmentation) de la température de traitement avec du monoxyde de carbone par rapport à la gamme de températures préconisée de 400 à 700°C ne permet pas d'obtenir le dégagement du carbone à la surface des particules ferromagnétiques ni, donc, la formation d'une surface possédant une activité d'adsorption, ce qui diminue l'activité biologique des matières physiologiquement actives.

La présente invention sera mieux comprise à la lecture de la description, qui va suivre, des exemples de réalisation du procédé d'obtention du support à commande magnétique porteur d'une matière physiologiquement active selon l'invention.

### EXEMPLE 1

100g de fer en poudre d'une granulométrie de 100 à 120 »m sont chauffés à la température de 800°C sous atmosphère de vapeur d'eau pendant 30 minutes, puis les particules de fer activées obtenues sont soumises au traitement avec du monoxyde de carbone à la température de 400°C dans un réacteur tubulaire hermétique, en réglant la température à ±5°C près. La teneur en carbone dégagé pendant le traitement de la poudre de fer activée sous atmosphère de monoxyde de carbone est contrôlée d'après la composition de la phase gazeuse à la sortie du réacteur. Ce traitement est effectué pendant 2 heures. 200 mg de particules de fer-carbone obtenues d'une capacité d'adsorption de 24 mg/g (pour le bleu de méthylène) sont placés dans une solution de 4 mg de daunorubicyne dans 10 ml d'eau distillé et le mélange obtenu est traité aux ultrasons à 22-44 kHz pendant 10 minutes, puis la dispersion soumise à la centrifugation, l'eau est évacuée et le résidu est séché. On obtient un support à commande magnétique sous forme d'une poudre noire finement dispersée, attirable par un aimant et se composant de fer à 43% en masse et de carbone pour le reste ; le teneur en daunorubicyne est de 2% en masse.

### EXEMPLE 2

100 g de fer en poudre d'une granulométrie de 300 à 500 »m sont chauffés à une température de 900°C sous atmosphère de vapeur d'eau pendant 30 minutes, puis les particules de fer activées ainsi obtenues sont soumises au traitement avec du monoxyde de carbone, comme indiqué dans l'exemple 1 à une température de 550°C pendant 5 heures. 200 mg de particules de fer-carbone obtenues, d'une capacité d'adsorption de 32,2 mg/g (pour le bleu de méthylène) sont placés dans une solution de 4 mg de carminomicyne dans 10 ml de solution de chlorure de sodium à 0,9%. Le mélange obtenu est agité pendant 10 minutes, puis centrifugé, l'eau est évacuée et le résidu séché. On obtient un support à commande magnétique sous forme d'une poudre noire finement dispersée, attirable par un aimant et se compose de 10% en masse de fer de carbone pour le reste ; teneur en carminomicyne : 2,0% en masse.

### EXEMPLE 3

100 g de fer en poudre d'une granulométrie de 100 à 200 »m sont chauffés à une température de 1100°C sous atmosphère d'air pendant 30 minutes. Le traitement des particules activées obtenues avec du monoxyde de carbone est effectué comme dans l'exemple 1 à la température de 600°C pendant 40 minutes. 200 mg de particules de fer-carbone obtenues, d'une capacité d'adsorption de 6 mg/g (pour le bleu de méthylène), sont placés dans une solution de 4 mg de doxorubicyne dans l'eau distillée. Le mélange obtenu est agité pendant 10 minutes, puis centrifugé, l'eau est évacuée et le résidu est séché. Le support à commande magnétique ainsi obtenu est une poudre noire attirable par un aimant et se compose de fer à 90% en masse et de carbone pour le reste ; la teneur en doxorubicyne est de 0,5% en masse.

### EXEMPLE 4

100 g de fer en poudre d'une granulométrie de 300 à 500 »m sont chauffés à une température de 1000°C sous atmosphère d'air pendant 30 minutes. Les particules de fer activées ainsi obtenues sont traitées avec du monoxyde de carbone comme indiqué dans l'exemple 1 à la température de 500°C pendant 90 minutes. 40 mg de particules de fer-carbone obtenues, d'une capacité d'adsorption de 19,5 mg/g (pour le bleu de méthylène) sont placés dans une solution de 25000 UI de célyase dans 1 ml de solution de chlorure de sodium à 0,9%. Le mélange obtenu est traité aux ultra-sons (22 à 44 kHz) pendant 15 minutes, puis centrifugé, l'eau est évacuée et le résidu est séché. Le support à commande magnétique obtenu est une poudre noire finement dispersée, attirable par un aimant et se compose de fer à 60% en masse et de carbone pour le reste ; la teneur en célyase est de 500 UI par 1 mg de support.

### EXEMPLE 5

100 g de fer en poudre d'une granulométrie de 100 à 200 »m sont chauffés à une température de 1200°C sous atmosphère d'air pendant 30 minutes. Les particules de fer activées ainsi obtenues sont traitées avec du monoxyde de carbone comme indiqué dans l'exemple 1 à la température de 700°C pendant 2 heures. 20 mg de particules de fer-carbone obtenus d'une capacité d'adsorption de 18,0 mg/g (pour le bleu de méthylène), sont placés dans une solution à 0,6 mg de diphénylhydramine dans 1 ml d'eau distillée. Le mélange obtenu est traité aux ultra-sons (22 à 44 kHz) pendant 20 minutes, puis centrifugé, l'eau est évacuée et le résidu est séché. Le support à commande magnétique ainsi obtenu est une poudre noire finement dispersée, attirable par un aimant et se compose de fer à 60 % en masse et de carbone pour le reste ; la teneur en diphénylhydramine est de 3 % en masse.

### Exemple 6

100 g de fer en poudre d'une granulométrie de 300 à 500 »m sont chauffés à une température de 1000°C sous atmosphère d'air pendant 30 minutes. Les particules de fer activées obtenues sont traitées avec du monoxyde de carbone comme indiqué dans l'exemple 1 à une température de 500°C pendant 15 heures. 200 mg de particules de fer-carbone d'une capacité d'adsorption de 19,5 mg/g (pour le bleu de méthylène) sont placés dans une solution à 5 mg de chloramphénicol dans 10 ml d'eau distillée. Le mélange obtenu est agité pendant 20 minutes, puis centrifugé, l'eau est évacué et le résidu est séché. Le support à commande magnétique obtenu est une poudre noire finement dispersée, attirable par un aimant et se compose de fer à 60 % en masse et de carbone pour le reste ; la teneur en chloramphénicol est de 2,5 % en masse.

### Exemple 7

200 mg de particules de fer-carbone obtenues comme indiqué dans l'exemple 6 sont placés dans une solution à 5 mg de doxorubicyne dans 10 ml d'eau. Le mélange obtenu est agité pendant 15 minutes, puis centrifugé, l'eau est évacuée et le résidu est séché. Le support à commande magnétique obtenu est une poudre noire finement dispersée, attirable par un aimant et se compose de fer à 60 % en masse et de carbone pour le reste ; la teneur en doxorubicyne est de 2,5 % en masse.

### Applicabilité industrielle

Le support de matières physiologiquement actives selon l'invention peut être utilisé dans la médecine pour le traitement de diverses maladies caractérisées par un foyer pathologique localisé dans lequel le support, selon l'invention, est maintenu à l'aide d'un champ magnétique permanent à fort gradient créé dans la zone de ce foyer. On peut citer parmi ces maladies les tumeurs malignes et bénignes, la thromboembolie ou les inflammations septiques et réactionnelles.

Le support selon l'invention peut être utilisé dans des buts de diagnostic. Dans ce cas les matières physiologiquement actives importantes au point de vue du diagnostic sont adsorbées "in vitro" ou "in vivo" par les particules de fer-carbone, les particules sont concentrées à l'aide d'un champ magnétique et ces matières sont ensuite soumies à l'analye après désorption.

Le support selon l'invention peut également être utilisé pour l'extraction des matières physiologiquement actives toxiques ou nuisibles contenues, par exemple dans le sang ou l'eau potable, grâce à leur adsorption par les particules de fer-carbone qui sont ensuite supprimées dans le liquide à épurer, à l'aide de champ magnétique permanent.

## Revendications

1. Support à commande magnétique porteur d'une matière physiologiquement active, caractérisé en ce qu'il est réalisé sous forme de particules de fer-carbone composées de fer à 10-90% en masse et de carbone pour le reste, ayant une capacité d'adsorption d'au moins 6 mg/g pour le bleu de méthylène et portant sous forme adsorbée la matière physiologiquement active.

2. Support à commande magnétique porteur d'une matière physiologiquement active selon la revendication 1, caractérisé en ce qu'il comprend des particules de fer-carbone composées de fer à 50-70% en masse et de carbone pour le reste et en ce qu'il comporte en tant que matière physiologiquement active un antibiotique anticancéreux anthracyclinique.

3. Support à commande magnétique porteur d'une matière physiologiquement active selon la revendication 2, caractérisé en ce que l'antibiotique anticancéreux anthracyclynique est choisi parmi la daunorubicine, la doxorubicine et la carminomycine.

4. Support à commande magnétique porteur d'une matière physiologiquement active selon la revendication 1, caractérisé en ce qu'il comprend des particules de fer-carbone composées de fer à 50-70% en masse et de carbone pour le reste et en ce qu'il comporte en tant que matière physiologiquement active une enzyme dans une proportion de 500 UI par 1 mg de particules de fer-carbone.

5. Support à commande magnétique porteur d'une matière physiologiquement active selon la revendication 4, caractérisé en ce que l'enzyme est la célyase.

6. Support à commande magnétique porteur d'une matière physiologiquement active selon la revendication 1, caractérisé en ce qu'il comprend des particules de fer-carbone composées de fer à 50-70% en masse et de carbone pour le reste et en ce qu'il comporte en tant que matière physiologiquement active un médicament antihistaminique dans une proportion allant de 2 à 3% en masse.

7. Support à commande magnétique porteur d'une matière physiologiquement active selon la revendication 6, caractérisé en ce que le médicament antihistaminique est la diphénylhydramine.

8. Procédé d'obtention du support à commande magnétique porteur d'une matière physiologiquement active selon la revendication 1, caractérisé en ce que le fer en poudre est chauffé sous atmosphère oxydante à une température de 800 à 1200°C, puis traité avec du monoxyde de carbone à une température de 400 à 700°C, pour obtenir des particules de fer-carbone qui sont ensuite placées dans une solution de matières physiologiquement actives, où ces matières sont adsorbées à la surface desdites particules.

9. Procédé selon la revendication 8, caractérisé en ce qu'on utilise du fer en poudre d'une granulométrie de 100 à 500 »m.

10. Procédé selon la revendication 8 ou 9, caractérisé en ce qu'on utilise la vapeur d'eau ou l'air comme atmosphère oxydante.

## Claims

1. A magnetically-controlled carrier for a physiologically active material, characterized in that it is provided in the form of iron-carbon particles comprising 10 to 90% by weight of iron the remainder being carbon, having an adsorption capacity for methylene blue of at least 6 mg/g and carrying the physiologically active substances in an adsorbed form.

2. A magnetically-controlled carrier for a physiologically active substance according to claim 1, characterized in that it comprises particles of iron-carbon comprising 50-70% by weight of iron the remainder being carbon, and in that it includes as a physiologically active substance an anthracyclinic anti-cancer antibiotic.

3. A magnetically-controlled carrier for a physiologically active substance according to claim 2, characterized in that the anthracyclynic anti-cancer antibiotic is selected from daunorubicin, doxorubicin and carminomycin.

4. A magnetically-controlled carrier for a physiologically active substance according to claim 1, characterized in that it comprises particles of iron-carbon comprising 50-70% by weight of iron, the remainder being carbon, and in that it includes as by physiologically active substance an enzyme in a proportion of 500 IU per mg of iron-carbon particles.

5. A magnetically-controlled carrier for a physiologically active substance according to claim 4, characterized in that the enzyme is celyase.

6. A magnetically-controlled carrier for a physiologically active substance according to claim 1, characterized in that it comprises particles of iron-carbon comprising 50-70% by weight of iron, the remainder being carbon, and in that it includes as the physiologically active substance an anti-histamin medicament in an amount of 2 to 3% by weight.

7. A magnetically-controlled carrier for a physiologically active substance according to claim 6, characterized in that the anti-histamin medicament is a diphenyl-hydramine.

8. Method for obtaining a magnetically-controlled carrier for a physiologically active substance according to claim 1, characterized in that iron in powder form is heated in an oxidizing atmosphere at a temperature à 800 to 1200°C and then heated with a carbon monoxide at a temperature of 400 to 700°C in order to obtain iron-carbon particles which are then placed in a solution of physiologically active substances where said substances are adsorbed at the surface of said particles.

9. Method according to claim 8, characterized in that the iron in powder form has a particle size of 100 to 500 »m.

10. Method according to claim 8 or 9, characterized in that water vapor or air is employed as the oxidizing atmosphere.

## Patentansprüche

1. Magnetkontrollierbarer Träger mit physiologisch aktivem Mittel,
**dadurch gekennzeichnet,**
daß er in Form von Teilchen aus Eisen-Kohlenstoff hergestellt ist, die aus 10 bis 90 Massenprozent Eisen und aus Kohlenstoff als restlichem Bestandteil zusammengesetzt sind, eine Adsorptionskapazität von mindestens 6 mg Methylenblau/g aufweisen und in ihrer adsorbierten Form physiologisch aktive Mittel tragen.

2. Magnetkontrollierbarer Träger mit physiologisch aktivem Mittel nach Anspruch 1,
**dadurch gekennzeichnet,**
daß er Teilchen aus Eisen-Kohlenstoff umfaßt, die zu 50 bis 70 Massenprozent aus Eisen und aus Kohlenstoff als restlichem Bestandteil zusammengesetzt sind, und daß er als physiologisch aktives Mittel ein gegen Krebs wirkendes Anthracyclin-Antibiotikum umfaßt.

3. Magnetkontrollierbarer Träger mit physiologisch aktivem Mittel nach Anspruch 2,
**dadurch gekennzeichnet,**
daß das gegen Krebs wirkende Anthracyclin-Antibiotikum ausgewählt ist aus Daunorubicin, Doxorubicin und Carminomycin.

4. Magnetkontrollierbarer Träger mit physiologisch aktivem Mittel nach Anspruch 1,
**dadurch gekennzeichnet,**
daß er Teilchen aus Eisen-Kohlenstoff umfaßt, die zu 50 bis 70 Massenprozent aus Eisen und aus Kohlenstoff als restlichem Bestandteil zusammengesetzt sind, und daß er als physiologisch aktives Mittel ein Enzym in einem Anteil von 500 internationalen Einheiten auf 1 mg der Teilchen aus Eisen-Kohlenstoff umfaßt.

5. Magnetkontrollierbarer Träger mit physiologisch aktivem Mittel nach Anspruch 4,
**dadurch gekennzeichnet,**
daß das Enzym Célyase ist.

6. Magnetkontrollierbarer Träger mit physiologisch aktivem Mittel nach Anspruch 1,
**dadurch gekennzeichnet,**
daß er Teilchen aus Eisen-Kohlenstoff umfaßt, die zu 50 bis 70 Massenprozent aus Eisen und aus Kohlenstoff als restlichem Bestandteil zusammengesetzt sind, und daß er als physiologisch aktives Mittel ein Antihistaminarzneimittel in einem Anteil entsprechend 2 bis 3 Massenprozent umfaßt.

7. Magnetkontrollierbarer Träger mit physiologisch aktivem Mittel nach Anspruch 6,
**dadurch gekennzeichnet,**
daß das Antihistaminarzneimittel Diphenylhydramin ist.

8. Verfahren zur Herstellung eines magnetkontrollierbaren Trägers mit physiologisch aktivem Mittel nach Anspruch 1,
**dadurch gekennzeichnet,**
daß das Eisenpulver in einer oxidierenden Atmosphäre auf eine Temperatur von 800 bis 1200°C erhitzt wird, dann mit Kohlenmonoxid bei einer Temperatur von 400 bis 700°C behandelt wird, um Teilchen aus Eisen-Kohlenstoff zu erhalten, die dann in eine Lösung aus physiologisch aktiven Mitteln eingebracht werden, wo die Mittel auf der Oberfläche der Teilchen adsorbiert werden.

9. Verfahren nach Anspruch 8,
**dadurch gekennzeichnet,**
daß es Eisenpulver mit einer Korngröße von 100 bis 500 »m verwendet.

10. Verfahren nach Anspruch 8 oder 9,
**dadurch gekennzeichnet,**
daß es Wasserdampf oder Luft als oxydierende Atmosphäre verwendet.
